# EUROPEAN PATENT SPECIFICATION

(11) **EP 2 156 185 B1**
(45) Date of publication and mention of the grant of the patent: **14.08.2013**
(21) Application number: 08758211.0
(22) Date of filing: 02.06.2008
(51) Int. Cl.: G01N 33/53, G01N 33/543, G01N 33/553

(54) **BIO SURFACE ACOUSTIC WAVE (SAW) RESONATOR AMPLIFICATION WITH NANOPARTICLES FOR DETECTION OF A TARGET ANALYTE**
BIOOBERFLÄCHENWELLENRESONATOR, NACHWEIS EINES ZIELANALYTEN UNTER AMPLIFIZIERUNG MIT HILFE VON NANOPARTIKELN
AMPLIFICATION D'UN RÉSONATEUR D'ONDES ACOUSTIQUES DE SURFACE (SAW) BIOLOGIQUE AVEC DES NANOPARTICULES POUR LA DÉTECTION D'UN ÉLÉMENT CIBLE À ANALYSER.

(30) Priority: 01.06.2007 DK 200700805
(43) Date of publication of application: 24.02.2010
(73) Proprietor: Atonomics A/S, 2450 Copenhagen SV (DK)
(72) Inventor: WARTHOE, Peter, DK-2100 Copenhagen O (DK)
(74) Representative: Bender, Mikkel
(86) International application number: PCT/DK2008/000202
(87) International publication number: WO 2008/145130

(56) References cited:
- WO-A-2008/019693
- US-A- 5 501 986
- CHU ET AL: "Quartz crystal microbalance immunoassay with dendritic amplification using colloidal gold immunocomplex" SENSORS AND ACTUATORS B, ELSEVIER SEQUOIA S.A., LAUSANNE, CH, vol. 114, no. 2, 26 April 2006 (2006-04-26), pages 696-704, XP005349362 ISSN: 0925-4005
- MAO ET AL: "A nanoparticle amplification based quartz crystal microbalance DNA sensor for detection of Escherichia coli O157:H7" BIOSENSORS & BIOELECTRONICS, ELSEVIER SCIENCE PUBLISHERS, BARKING, GB, vol. 21, no. 7, 15 January 2006 (2006-01-15), pages 1178-1185, XP005215700 ISSN: 0956-5663
- NIE L B ET AL: "Enhanced DNA detection based on the amplification of gold nanoparticles using quartz crystal microbalance" NANOTECHNOLOGY; NANOTECHNOLOGY AUG 8 2007, vol. 18, no. 30, 8 August 2007 (2007-08-08), XP002493546
- GAO Z ET AL: "Detection of nucleic acids using enzyme catalysed template guided deposition of Polyaniline" ADVANCED MATERIALS, WILEY VCH, WEINHEIM, DE, vol. 19, 25 January 2007 (2007-01-25), pages 602-606, XP002474944 ISSN: 0935-9648
- CHU X ET AL: "Silver-enhanced colloidal gold metalloimmunoassay for Schistosoma japonicum antibody detection" JOURNAL OF IMMUNOLOGICAL METHODS, ELSEVIER SCIENCE PUBLISHERS B.V.,AMSTERDAM, NL, vol. 301, no. 1-2, 1 June 2005 (2005-06-01), pages 77-88, XP004976652 ISSN: 0022-1759

## Description

### Technical Field

The present invention relates generally to a signal amplification method for a SAW resonator microsensor for analyzing test samples, containing target analyte including proteins and nucleic acids. The method finds use in numerous chemical, environmental and medical applications.

The method comprises the steps: reacting the analyte in a sample with a first molecular recognition component and a second molecular recognition component linked to at least one nanoparticle; and adding an enhancement solution comprising silver ions and/or gold ions, and a reducing agent, whereby the silver ions and/or gold ions are reduced to metallic silver and/or gold which is deposited onto the surface of the at least one nanoparticle; wherein the mass increase of the at least one nanoparticle is detected by a SAW sensor.

### Background

Sensitive detection of analyte, such as biological analyte, continues to be a significant challenge in analytical detection methods. Frequently, detection methods require processing of multiple samples. In addition, analytical detection methods should be easy, rapid, and reproducible. This is particularly important when highly specialized methods and reagents, such as diagnostic methods, are unavailable.

Conventional bioanalytical methods in particular have several deficiencies. For example, hybridization of nucleic acid molecules is generally detected by autoradiography or phosphor image analysis when the hybridization probe contains a radioactive label or by densitometer when the hybridization probe contains a label, such as biotin or di-goxin. The label can in turn be recognized by an enzyme-coupled antibody or ligand. Most modern biomolecule detection methods require modification of the molecule e.g. DNA or RNA or protein, making current detection methods expensive and labor intensive.

Acoustic wave sensor technology has shown broad application in detecting materials.

Acoustic wave sensors detect materials by generating and observing an acoustic wave. As the acoustic wave propagates through or on the surface of the material, any changes to the characteristics of the propagation path affect the velocity and/or amplitude of the wave. The amplitude, frequency, and/or phase characteristics of the sensor can be measured and correlated to a corresponding physical quantity.

Several different types of acoustic wave devices have been developed, but all have only limited success in measuring water soluble or biological samples. Bulk acoustic waves (BAW) propagate through a medium. The most commonly used BAW devices are the thickness shear mode (TSM) resonator most comment types are quartz crystal microbalances and the shear-horizontal acoustic plate mode (SH-APM) sensor. Conversely, waves that propagate on the surface of the substrate are known as surface waves. The most widely used surface wave devices are the surface acoustic wave sensor and the shear-horizontal surface acoustic wave (SH-SAW) sensor, also known as the surface transverse wave (STW) sensor. All acoustic wave sensors will function in gaseous or vacuum environments, but very few of them will operate efficiently when they are in contact with liquids.

Of the known acoustic sensors for liquid sensing, the Love wave sensor, a special class of the shear-horizontal SAW, has the highest sensitivity. To make a Love wave sensor, a dielectric waveguide coating is placed on a SH-SAW device such that the energy of the shear horizontal waves is focused in that coating. A biorecognition coating is then placed on the waveguide coating, forming the complete *biosensor.* Successful detection of anti-goat IgG in the concentration range of ng/ml using a 110 MHz YZ-cut SH-SAW with a polymer Love wave guide coating has been achieved [E. Gizeli et al. 1997. "Antibody Binding to a Functionalized Supported Lipid Layer: A Direct Acoustic Immunosensor," Anal Chem, Vol. 69:4808-4813.].

A comparison between different SAW sensors has recently been described [Bio-molecular Sensors, Eds. Electra Gizeli and Christoffer R. Lowe (2002)]. They describes a 124 MHz Love wave sensor have a sensitivity of 1.92 mg/cm2. The use of SAW sensors for detection of biological compounds have been reported in, for example, U.S. 5,478,756, W09201931 and WO03019981, each of which is incorporated herein by reference in its entirety.

Conventional SAW devices are a poor choice for liquid detection, as the vertical component of the propagating wave is suppressed by the liquid-air barrier. One acoustic wave sensor that functions in liquids is a shear-horizontal SAW sensor. If the cut of the piezoelectric crystal material is rotated appropriately, waves propagate horizontally and parallel to a liquid surface. This dramatically reduces loss when liquids come into contact with the propagating medium, allowing the SH-SAW sensor to operate as a *biosensor.* Many efforts at detecting liquid solution analytes (such as biological molecules) have focused on defining the interaction between the acoustic wave and the properties of the solid/liquid interface, as well as designing higher frequency SAW devises operating in the GHz range.

The present application provides a solution to the inability of SAW devices to measure analytes, including biomolecules, in liquids.

The use of SAW devices in immunoassays has been described previously. These devices consist of single crystal wafers sandwiched between two electrodes. The electrodes are provided with means for connecting these devices to an external oscillator circuit that drives the quartz crystal at its resonant frequency. This frequency is dependent on the mass of the crystal, as well as the mass of any layers confined to the electrode areas of the crystal. Thus, the frequency is altered by changes in mass on the surface of the electrodes or in any layers on those electrodes. In general, the change in resonant frequency of these devices can be correlated to the amount of mass change.

US 4,235,983, issued to Rice on Dec. 2, 1980, discloses a method for the determination of a particular subclass of antibody. The method utilizes a piezoelectric oscillator having bound to its two dimensional surface an antigen specific for the antibody to be determined. The antigen-coated oscillator is exposed to a solution containing an unknown amount of the antibody. After the antibody in the solution is attached to the antigen on the oscillator, the oscillator is exposed to a so-called sandwiching substance which selectively binds to a specific subclass of the antibody being determined. The frequency of the oscillator is measured in the dry state before and after exposure to the sandwiching substance. The change in frequency is related to the amount of the subclass of antibody bound to the two dimensional oscillator surfaces and the amount of the subclass of antibody in the solution can be determined by reference to a standard curve.

Roederer et al. disclose an in-situ immunoassay using piezoelectric quartz crystals, specifically, surface acoustic wave devices. Goat anti-human IgG was immobilized on the two dimensional quartz crystal surface with a coupling agent. The piezoelectric crystals were then placed in an electric oscillator circuit and tested for detection of the antigen human IgG. Detection was based upon the fact that surface mass changes by adsorption are reflected as shifts in the resonant frequencies of the crystals The authors concluded that the method suffers from both poor sensitivity and poor detection limits. The authors also concluded that the antigen to be detected must be of high molecular weight; low molecular weight analytes cannot be directly detected by this methodology. [Analytical Chemistry, Vol. 55, (1983)].

Ngeh-Ngwainbi et al. describe the use of piezoelectric quartz crystals coated with antibodies against parathion which are used for the assay of parathion in the gas phase. When the coated antibody binds with parathion by a direct reaction in the gas phase, the resulting mass change on the crystal generates a frequency shift proportional to the concentration of the pesticide. [J. Mat. Chem. Soc., Vol. 108, pp. 5444-5447 (1986)].

US patent No. 4,999,284, issued to Ward on March 12, 1991, discloses a method using a quartz crystal microbalance assay in which the binding of analyte to a surface on or near a quartz crystal microbalance (QCM) is detected by a conjugate which comprises an enzyme capable of catalyzing the conversion of a substrate to a product capable of accumulating on or reacting with a two dimensional surface of the QCM leading to a mass change and, hence, a change in resonant frequency. However the frequency was only 406Hz changes in 30 minutes at a concentration of 0.24 ng/ml and 6.3 Hz changes in 30 minutes at a concentration of 0,002 ng/ml (2 pg/ml) of the analyte APS reductase using an anti-APS reductase antibody. Using different modification of the two dimensional QCM surface the author succeeded to obtaining 22 Hz changes in 30 minutes at a concentration of 0.025 ng/ml (25 pg/ml) of the analyte. Those result indicated that at very low concentration (pg/ml range) the delta Hz changes is down if not under the detection/noise level.

In general piezoelectric based immunoassays in which mass change is attributable to the immunological reaction between an antigen and an antibody can under circumstances where a two dimensional sensor surfaces are used, suffer from poor sensitivity and poor detection limit. Consequently, there is a need in the art for a piezoelectric based specific binding assay in which the reaction between a molecular recognition component and its target analyte can be amplified to provide a more sensitive assay.

### Disclosure of the Invention

The inventor has discovered that changing the liquid/solid volume ratio in three-dimensional micro channels structures between IDTE structures or reflector structures on a piezoelectric substrate significant changes the frequency and phase of the system. The changes of the liquid/solid volume ratio between said micro channels can be directed correlated to the analyte concentration in a test sample

The invention related to a microsensor for detecting the presence of a target analyte in a test sample solution comprising; at least one but preferable two or more surface acoustic wave (SAW) resonator units each comprising; a piezoelectric substrate; a plurality of interdigital transducer electrode (IDTE) and reflectors on a surface of said substrate, wherein three-dimensional micro channels are formed between said electrodes and reflectors; wherein said SAW resonator unit having at least one molecular recognition component immobilized in the micro channel formed between said IDTE structures and reflector structures; reacting the target analyte in the test sample with at least one molecular recognition component; further reacting with a seconds enzyme linked molecular recognition component or enzyme linked analyte; further reacting with a substrate which is converted into a insoluble precipitates which accumulates in the micro channels formed between said electrode and reflector structures and thereby decreases the solid /liquid volume ratio in said micro channels; said solid /liquid volume ratio changes in said micro channels leading to a signal change of frequency or phase.

For the SAW resonator sensor types, stiffness changes in the biofilm in the three-dimensional micro channels between the IDTE and reflector structures also either increase or decrease the frequency of the SAW resonator unit depending of the setup. This phenomena using hydrogel technology have been described elsewhere in US application 20060024813 by same author.

The invention relates to a method for detecting a target analyte in a sample, said method comprising the steps:
(a) reacting the analyte in a sample with a first molecular recognition component and a second molecular recognition component linked to at least one nanoparticle; and
(b) adding an enhancement solution comprising silver ions and/or gold ions, and a reducing agent, whereby the silver ions and/or gold ions are reduced to metallic silver and/or gold which is deposited onto the surface of the at least one nanoparticle; and
(c) detecting the at least one nanoparticle comprising deposited silver ions and/or gold ions by a SAW sensor.

Preferably, the nanoparticle is a gold particle. Preferably, the nanoparticle has a diameter from 0.05 nm to 20 nm. in preferred embodiments the nanoparticle has a diameter of 0,05; 0,1; 0,2; 0,3; 0,4; 0,5; 0,6; 0,7; 0,8; 0,9; 1; 2; 3; 4; 5; 6; 7; 8; 9; 10; 11; 12; 13; 14; 15; 16; 17; 18; 19 or 20 nm. An about 10 nm size nanoparticle increases in diameter to about 300 nm within 5 minutes upon deposit of precipitate according to the method of the invention.

The invention further relates to a kit of parts comprising a microsensor for detecting an analyte in a sample, comprising:
(a) a Surface Acoustic Wave sensor; said Surface Acoustic Wave sensor comprising at least one first molecular recognition component immobilized to the surface of the SAW sensor;
(b) at least one second molecular recognition component linked to at least one nanoparticle;
(c) an enhancement solution comprising silver ions and/or gold ions; and (d) a reducing agent.

The present application is directed to microsensors for detecting the presence of a target analyte in a sample solution. The microsensor includes a SAW resonator sensor having three-dimensional micro channels structures on the sensor surface. The micro channel structures further includes an immobilized molecular recognition component that is capable of binding the target analyte. the target analyte further react, preferably in the micro channels, with a seconds enzyme linked molecular recognition component or enzyme linked target analyte; further reacting with a substrate which is converted into a insoluble precipitates which accumulates in the micro channels formed between said electrode and reflector structures and thereby decreases the solid /liquid volume ratio in said micro channels; said solid /liquid volume ratio changes in said micro channels leading to a signal change of frequency or phase.

The microsensor can also include a reference surface acoustic wave resonator micro channel structures. For example, the reference micro channels do not contain the molecular recognition component. Alternatively, the control can be a measure of a sample solution that does not comprise the target analyte. The difference between the signal of the micro reference channels and the sensor micro channels determines the presence of the target analyte.

Non-limiting examples of molecular recognition components include nucleic acids, nucleotides, nucleosides, nucleic acid analogues such as PNA and LNA molecules, proteins, peptides, antibodies including IgA, IgG, IgM, IgE, enzymes, enzymes cofactors, enzyme substrates, enzymes inhibitors, receptors, ligands, kinases, Protein A, Poly U, Poly A, Poly lysine, triazine dye, boronic acid, thiol, heparin, polysaccharides, coomassie blue, azure A, metal-binding peptides, sugar, carbohydrate, chelating agents, prokaryotic cells and eukaryotic cells.

Non-limiting examples of target analytes include nucleic acids, proteins, peptides, antibodies, enzymes, carbohydrates, chemical compounds, and gasses. Other exemplary target analytes include Troponin 1, Troponin T, allergens, or immunoglobulins such as IgE. In certain applications, the target analyte is capable of binding more than one molecular recognition component.

The present application is also directed to methods of detecting a target analyte in a sample solution. A molecular recognition component is immobilized in the micro channels located on a surface of a surface acoustic wave sensor. The sensor is contacted with the sample under conditions promoting binding of the analyte in the sample to the recognition component. A change in phase shift or frequency of a surface acoustic wave is then detected. The change determines the presence of the analyte in the sample.

It has been observed that the amplitude of the SAW resonator unit should be adjusted for optimal conditions for at least one molecular recognition component to react with the target analyte in the test sample. If the amplitude is too high inconsistent result can be obtained due to non optimal condition for molecular recognition component/analyte interaction.

A method for detecting an analyte in a test sample comprising the following steps; reacting the analyte in a test sample with a first recognition component and a second enzyme-linked recognition component of enzyme-linked analyte; adding a substrate which is converted into a insoluble precipitate by said linked enzyme; wherein, the two steps are executed in a reaction vessel segregate from the SAW resonator units, and wherein said insoluble precipitate obtained is actively transported to said SAW resonator units, where it upon contact with the SAW micro channels formed between said electrode and reflector structures decreases the solid /liquid volume ratio in said micro channels; said solid /liquid volume ratio changes in said micro channels leading to a signal change of frequency or phase, said signal change elicit an analyte specific signal.

A target analyte can be detected in any sample. Exemplary samples include blood, serum, plasma, ascites, faeces, spinal core fluids, urine, smears and saliva.. The method can be used for diagnostic purposes.

### Brief Description of the Drawings

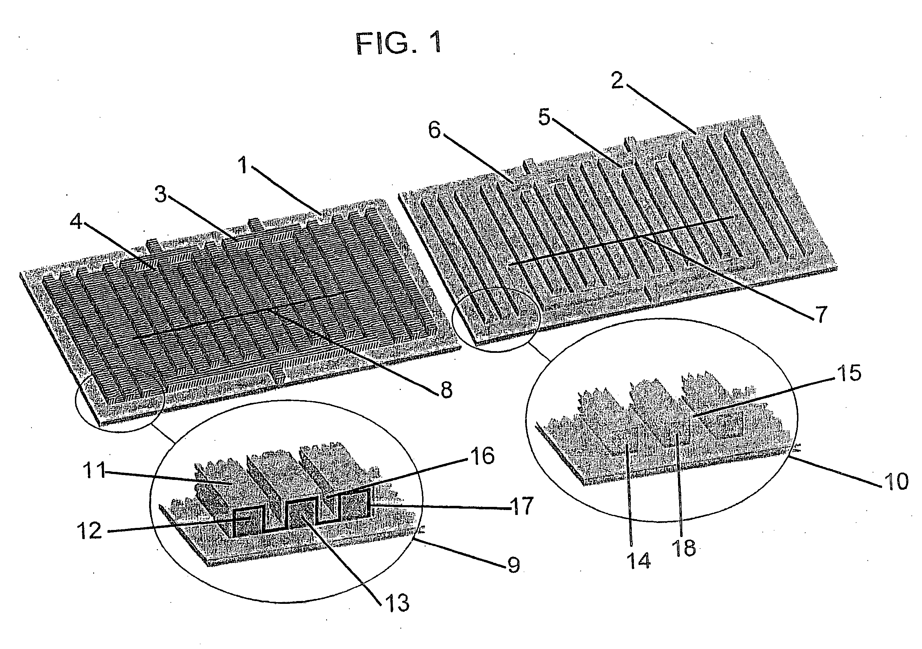

The invention is explained in detail below with reference to the drawings, in which
Fig. 1 illustrates two SAW resonator units indicated generally by the reference numeral 1 and 2. Each SAW resonator units consist of one IDTE part (7, 8) and two reflector parts (9, 10). Between the IDTE (3, 6) are located the micro channels (4, 5). Identical micro channels are also located between the reflector structures (15, 16). On the entire SAW resonator unit (1) three-dimensional surfaces, molecular recognition components are immobilized (3, 17), whereas on SAW resonator unit (2) no molecular recognition component is presence. The reaction layer (11) representing the analyte + second enzyme linked molecular recognition component + substrate. When the substrate converts to insoluble precipitates the micro channels space (16) between reflector structures (12, 13) decreases due to solid /liquid volume ratio changes in the micro channels (16). Also the micro channels between IDTE in SAW resonator unit (1) decreases for the same reasons. No micro channels (15) solid /liquid volume ratio changes are seen between the structures (14, 18) on the SAW resonator units (2).
Fig. 2 illustrates two SAW resonator units (1, 2) on the same substrate, otherwise identical to Fig. 1.
Fig. 3 illustrates a dose response curve further described under EXAMPLE 1.
Fig. 4 illustrates an AFM picture of the reflector structures illustrated in Fig. 1 (9). The (1) represent the substrate. Number 2 represent a reflector structures. Number 3, 4 represent the dimensional of the micro channel between the reflector structures.
Fig. 5 illustrates a calculation scheme from the AFM picture shown in Fig. 4. Here is given some examples of the dimensions of the IDTE and the reflector structures.

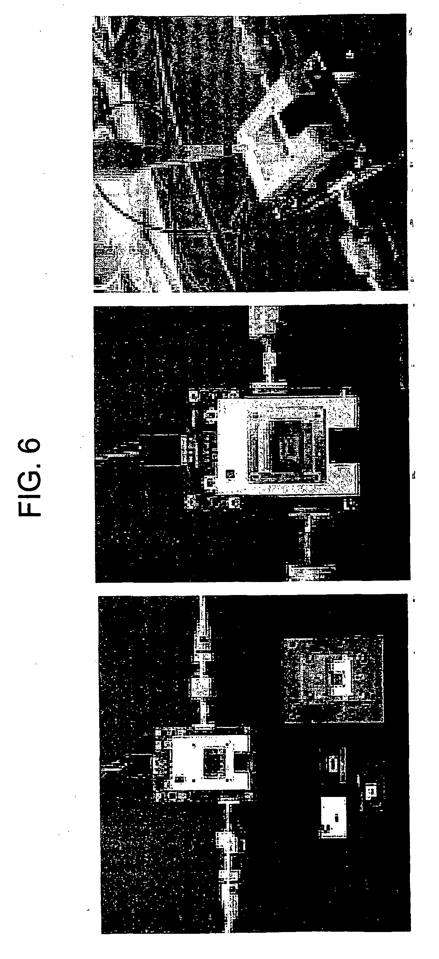

Fig. 6 illustrates a cartridge sealing for sensor; B: sensor in cartridge; C: isolation tape, D: assembling jig; E: electronic controlling unit; F; wires connection to computer; G: sensor including cartridge assembled into test setup; H: added the silver enhancing solution into sensor holes.
Fig. 7 illustrates the readout of the signal after adding Silver enhancement solution (procedure C4).
Fig. 8 illustrates a readout of the signal after adding BCIP/NBT solution (procedure C4).

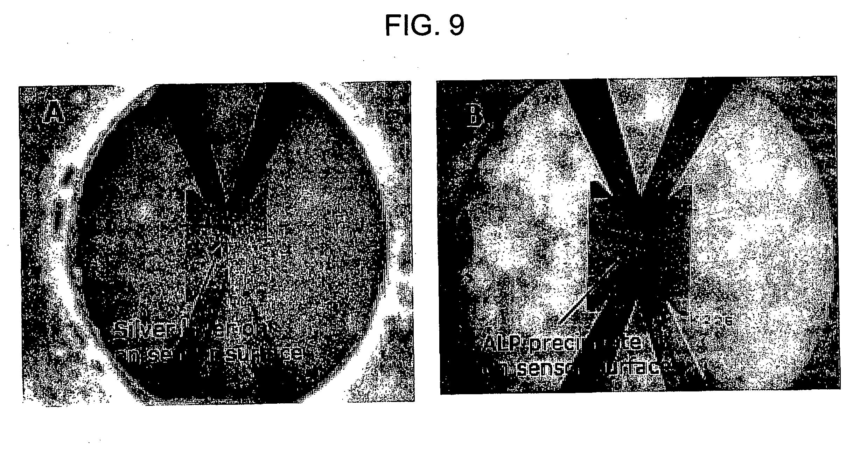

Fig. 9 illustrates a visually inspection of f2 sensor hole after sensor measurement. A: Gold conjugated antibodies and Silver enhancement method; B: Alkaline Phosphatase conjugated antibodies and BCIP/NBT method

### Detailed Description of the Invention

### Definitions

"Binding event" as used herein, means the binding of the target analyte to the molecular recognition component immobilized in the three-dimensional micro channel structure surface of the SAW sensor.

"Nanoparticle" is a solid insoluble particle which is preferably spherical and has a diameter of between 0.02 - 50 nm.

The invention may be understood by reference to the drawing(s) wherein like reference numerals are used to indicate like elements.

Referring now to Fig. 1, there is seen a micro sensor consisting of two SAW resonator units indicated generally by the reference numeral 1 and 2. Each SAW resonator units consist of one IDTE part (7, 8) and two reflector parts (9, 10). Between the IDTE (3, 6) are located the micro channels (4, 5). Identical micro channels are also located between the reflector structures (15, 16). On the entire SAW resonator unit (1) three-dimensional surfaces, molecular recognition components are immobilized (3, 17) whereas on SAW resonator unit (2) no molecular recognition component is presence. The reaction layer (11) representing the analyte + second enzyme linked molecular recognition component + substrate. When the substrate converts to insoluble precipitates the micro channels space (16) between reflector structures (12, 13) decreases due to solid /liquid volume ratio changes in the micro channels (16). Also the micro channels between IDTE in SAW resonator unit (1) decreases fro same reasons. No micro channels (15) solid /liquid volume ratio changes are seen between the structures (14, 18) on the SAW resonator units (2).

Examples of enzyme/substrate systems which are capable of producing an insoluble product which is capable of accumulating in the micro channels (16) include alkaline phosphatase and 5-bromo-4-chloro-3-indolylphosphate (BCIP). The enzymatically catalyzed hydrolysis of BCIP produces an insoluble dimer which precipitates in the micro channels. Other analogous substrates having the phosphate moiety replaced with such hydrolytically cleavable functionalities as galactose, glucose, fatty acids, fatty acid esters and amino acids can be used with their complementary enzymes.

Other enzyme/substrate systems include peroxidase enzymes, for example horseradish peroxidase (HRP) or myeloperoxidase, and one of the following: benzidene, benzidene dihydrochloride, diaminobenzidene, o-tolidene, o-dianisidine and tetramethyl-benzidene, carbazoles, particularly 3-amino-9-ethylcarbazole, all of which have been reported to form precipitates upon reaction with peroxidases. Also, oxidases such as alphahydroxy acid oxidase, aldehyde oxidase, glucose oxidase, L-amino acid oxidase and xanthine oxidase can be used with oxidizable substrate systems such as a phenazine methosulfate-nitriblue tetrazolium mixture.

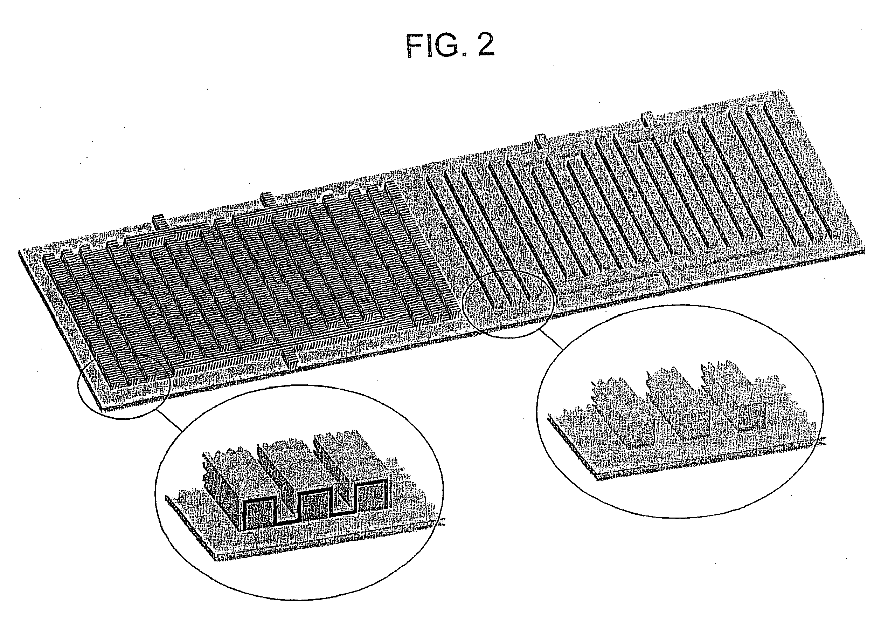

Referring now to Fig. 2, this Fig. 2 has two SAW resonator units (1,2) on the same substrate, otherwise identical to Fig. 1.

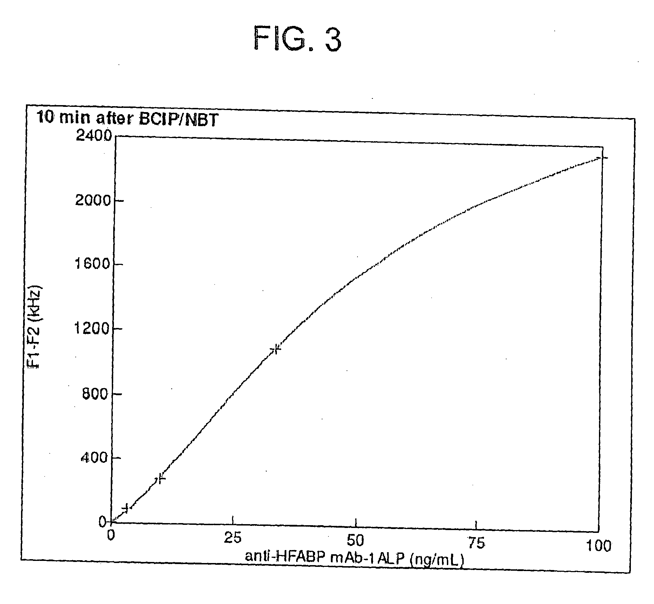

Referring now to Fig. 3, this Fig. 3 is further described under EXAMPLE 1.

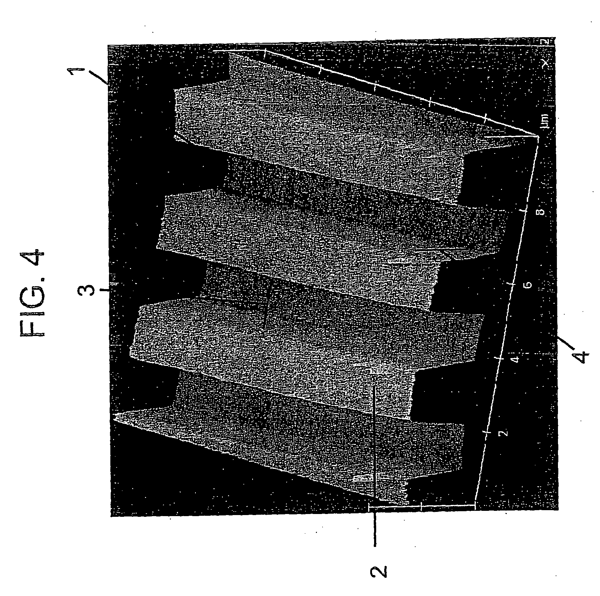

Fig. 4 depicts an AFM picture of the reflector structures illustrated in Fig 1. (9). The (1) represents the substrate. Number 2 represents a reflector structures. Numbers 3, 4 represent the dimensional of the micro channel between the reflector structures.

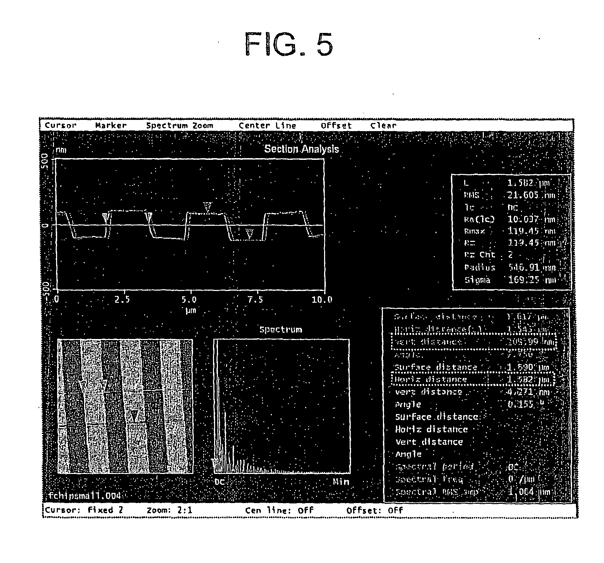

Fig. 5 depicts a calculation scheme from the AFM picture shown in Fig. 4. Here some examples of dimensions of the reflector and IDTE structures are illustrated.

### Surface Acoustic Wave Sensors

The microsensors disclosed herein include at least one surface acoustic wave sensor. A surface acoustic wave sensor includes a piezoelectric layer, or piezoelectric substrate, and input and output transducer(s). A surface acoustic wave is generated within the piezoelectric layer and is detected by interdigitated electrodes. As described in more detail below, binding events that alter the surface of the surface acoustic wave sensor can be detected as a change in a property of the propagating surface acoustic wave. Surface acoustic wave sensors are described in US patent Nos. 5,130,257; 5,283,037; and 5,306,644; F. Josse, et. al. "Guided Shear Horizontal Surface Acoustic Wave Sensors for Chemical and Biochemical Detection in Liquids," Anal. Chem. 2001, 73, 5937; and W. Welsch, et. al., "Development of a Surface Acoustic Wave Immunosensor," Anal. Chem. 1996, 68, 2000-2004; each of which is hereby expressly incorporated by reference in its entirety.

Acoustic wave devices are described by the mode of wave propagation through or on a piezoelectric substrate. Acoustic waves are distinguished primarily by their velocities and displacement directions. Many combinations are possible, depending on the material and boundary conditions. The interdigital transducer electrode (IDTE) of each sensor provides the electric field necessary to displace the substrate and thus form an acoustic wave. The wave propagates through the substrate, where it is converted back to an electric field at the IDTE at the opposing electrode. Transverse, or shear, waves have particle displacements that are normal to the direction of wave propagation and which can be polarized so that the particle displacements are either parallel to or normal to the sensing surface. Shear horizontal wave motion signifies transverse displacements polarized parallel to the sensing surface; shear vertical motion indicates transverse displacements normal to the surface..

"Surface acoustic wave sensor", or "surface acoustic wave device" as used herein mean any device that operates substantially in the manner described above. In some embodiments, "surface acoustic wave sensor" refers to both surface transverse wave devices, where the surface displacement is perpendicular to the direction of propagation and parallel to the device surface, as well as surface acoustic wave sensors where at least a portion of the surface displacement is perpendicular to the device surface. While surface transverse wave devices generally have better sensitivity in a fluid, it has been shown that sufficient sensitivity may also be achieved when a portion of the surface displacement is perpendicular to the device surface. See, for example, M. Rapp, et. al. "Modification of Commercially Available LOW-LOSS SAW devices towards an immunosensor for in situ Measurements in Water" 1995 IEEE International Ultrasonics Symposium, Nov. 7-10, 1995, Seattle, Wash.; and N. Barie, et. al., "Covalent bound sensing layers on surface acoustic wave biosensors," Biosensors & Bioelectronics 16 (2001) 979; all of which are expressly incorporated herein by reference.

The sensors are made by a photolithographic process. Manufacturing begins by carefully polishing and cleaning the piezoelectric substrate. Metal such as gold or aluminum is then deposited uniformly onto the substrate. The device is spin-coated with a photoresist and baked to harden it. It is then exposed to UV light through a mask with opaque areas corresponding to the areas to be metalized on the final device. The exposed areas undergo a chemical change that allows them to be removed with a developing solution. Finally, the remaining photoresist is removed. The pattern of metal remaining on the device is called an interdigital transducer (IDT) or interdigital electrodes (IDE). By changing the length, width, position, and thickness of the IDT, the performance of the sensor can be maximized.

### Input and Output Transducer(s)

The input and output transducers are preferably interdigitated transducers. Generally, there are two interdigital transducers. Each of the input and output transducers comprises two electrodes arranged in an interdigitated pattern. A voltage difference applied between the two electrodes of the input transducer results in the generation of a surface acoustic wave in the piezoelectric substrate. The electrodes generally may comprise any conductive material, with aluminum or gold being preferred.

The electrode(s) may take any conventional form but are preferably photolithographically deposited on the surface as elongate regions of metallisation transverse to the direction of propagation of a wave along the surface of the support. The elongate metallised regions preferably have a width and spacing of the same order of magnitude. The width is typically between 1 and 40 microns, preferably between 10 and 20 microns. In certain embodiments, the width is greater than or equal to 100 nm, 200 nm, 300 nm, 400 nm, 500 nm, 600 nm, 700 nm, 800 nm, 900 nm, 1 micron, 2 microns, 3 microns, 4 microns, 5 microns, 7.5 microns, 10 microns, 15 microns, 20 microns, 25 microns, 30 microns, 35 microns, 40 microns, 45 microns, 50 microns, 60 microns, 70 microns, 80 microns or 90 microns. In other embodiments, the space between the electrodes can be equal to or less than 100 microns, 90 microns, 80 microns, 70 microns, 60 microns, 50 microns, 45 microns, 40 microns, 35 microns, 30 microns, 25 microns, 20 microns, 15 microns, 10 microns, 7.5 microns, 5 microns, 4 microns, 3 microns, 2 microns 1 microns, 900 nm, 800 nm, 700 nm, 600 nm, 500 nm, 400 nm, 300 nm, 200 nm, 100 nm, or 75 nm. It should be noted that the spacing varies inversely with the frequency of the device.

In certain embodiments, the height of the electrodes is the same as the width of the electrodes. In other embodiments, the height of the electrodes is, for example, greater than or equal to 10 nm, 20 nm, 30 nm, 40 nm, 50 nm, 75 nm, 100 nm, 200 nm, 300 nm, 400 nm, 500 nm, 600 nm, 700 nm, 800 nm, or 900 nm. In other embodiments, the depth of the space between the electrodes can be less than or equal to 1 micron, 900 nm, 800 nm, 700 nm, 600 nm, 500 nm, 400 nm, 300 nm, 200 nm, 100 nm, 75 nm, 50 nm, 40 nm, 30 nm, or 20 nm.

In an alternative embodiment there is a single interdigital transducer. In this embodiment the single interdigital transducer, serves both as both an input and output transducer. In embodiments employing a single interdigital transducer acting as both input and output transducer, a reflector structure is generally provided to generate one or more resonances within the SAW sensor. The reflector structure may, for example, be a thin film grating. The grating may comprise aluminium, or another conductive material. The generated resonances can be detected, for example, by measuring the power dissipated at the single transducer. One or more binding events in the thin structure alter these resonances, allowing the binding events to be detected. An example of a sensor and technique according to this embodiment is generally described in US patent No. 5,846,708, hereby incorporated by reference. As described below, other electronics and/or circuitry may similarly be utilized in an embodiment employing a SAW sensor having only one interdigital transducer.

Molecular recognition molecules may be attached directly to self-assembled monolayers. For example, when gold IDTE's are employed, a DNA probe molecule may be attached using a SH group on the 5' of the DNA using self-assembled monolayers as known in the art and described, for example, in K. Vijayamohanan et al. "Self-assembled monolayers as a tunable platform for biosensor applications," Biosensors & Bioelectronics 17 (2002) 1-12 and George M. Whitesides et al. "Array of Self-Assembled Monolayers for studing inhibition of Bacterial Adhesion." Anal Chem 2002, 74, 1805-1810, both of which are hereby incorporated by reference.

The present invention especially relates to (1) a method for detecting an analyte in a sample comprising the following steps: (a) reacting the analyte in a sample with a first recognition component and a second enzyme-linked recognition component; and (b) adding a substrate which is converted into a precipitate by said linked enzyme; wherein, the steps (a) and (b) are executed in a reaction vessel segregate from the measuring chamber and wherein said precipitate obtained in step (b) is actively transported to said measuring chamber where it upon contact with the SAW sensor surface elicit an analyte specific signal.

Further embodiments of the present invention are:
(2) the method as the above (1) and/or as in one of the succeeding embodiments, wherein the first recognition component immobilizes the analyte to a surface in the reaction vessel.
(3) the method as the above (1) and/or as in one of the succeeding embodiments, wherein the surface may be the reaction vessel lining or the surface of material contained in the vessel cavity or a combination thereof.
(4) the method as the above (1) and/or as in one of the succeeding embodiments, wherein the recognition components is selected from the group containing: Protein, protein analogues, modified proteins, nucleic acid, nucleic acid analogues, modified nucleic acids.
(5) the method as the above (1) and/or as in one of the succeeding embodiments, wherein the protein is selected from the group containing: antibody, antibody fragments, modified antibodies, receptor molecules, ligand molecules.
(6) the method as the above (1) and/or as in one of the succeeding embodiments, wherein the enzyme is selected from the group containing: Alkaline phosphatase (AP), Horse radish peroxidase (HRP).
(7) the method as the above (1) and/or as in one of the succeeding embodiments, wherein wherein the substrate is selected from the group containing: diaminobenzidine (DAP), amino ethylcarbazole (AEC), Tetramethylbenzidine (TMB) or 5-bromo,4-chloro,3-indolylphosphate (BCIP) / nitroblue tetrazolium (NBT).

The present invention furthermore relates to (8) a device for detecting an analyte in a sample according to the method as the above (1) and/or as in one of the succeeding embodiments, comprising a reaction vessel with an inlet and an outlet in which the analyte specific precipitate is generated by enzyme conversion; connected to a measuring chamber with an inlet and an outlet comprising a SAW sensor; wherein, a liquid flow is actively transporting said precipitate from the reaction vessel to the measuring chamber where it comes into contact with the SAW sensor surface and elicits an analyte specific signal.
(9) the device as the above (8) and/or as in one of the succeeding embodiments, further connected to a flow regulator wherein said regulator is a pump system placed in front of the reaction vessel, a suction system placed after the measuring chamber or both.
(10) the device as the above (8) and/or as in one of the succeeding embodiments, wherein the reaction vessel is selected from the group containing: a tube, a tubing system, a chamber, a system of connected chambers.
(11) the device as the above (8) and/or as in one of the succeeding embodiments, further comprising material retained within the reaction vessel system selected from the group containing: beads, gel.
(12) the device as the above (8) and/or as in one of the succeeding embodiments, wherein the reaction vessel system further comprises one or more material selected from the group comprising: filter, grid.
(13) the device as the above (8) and/or as in one of the succeeding embodiments, wherein the SAW sensor is of SAW filter unit type.

The enzyme linked molecular recognition components may in some embodiments of the invention be substituted with nanoparticle linked molecular recognition components or nanoprobes.

An improvement of the signal amplitude has recently been invented by Atonomics. Until now Alkaline Phosphatase (ALP) conjugated antibodies have been used for signal generation on the oscillating SAW sensors. Several disadvantages have been identified using this ALP method such as:

ALP conjugated antibody binds unspecifically to the gold and resin wall inside the sensor cavity and on the surface of the substrate. Such binding creates non specific BCIP/NBT precipitate and eventually give rice to an increase of the CV value.

The BCIP/NBT precipitate in the SAW micro channels and on top of the SAW structures are not distributed as a plane layer all over the sensor structure (due to oscillating nature of the SAW sensor), but in a more random way on the sensor surface as illustrated at Fig. 4B - this also could give rice to increase of the CV value.

Since the Alkaline phosphatase method is an enzymatic process it can loose its activity over time due to temperature and handling issues. Also this could give rice to increase of the CV value.

To overcome such disadvantages Atonomics has initiated the test of nano-gold conjugated antibodies instead of Alkaline phosphatase conjugated antibodies.

From sensitivity point of view it seems likely that the gold method gives a 300X increased signal compared to our ALP method judged by Fig. 2 and Fig. 3.

Accordingly, in a preferred embodiments the invention relates to a method for detecting a target analyte in a sample comprising the steps: (a) Reacting the analyte in a sample with a first molecular recognition component and a second molecular recognition component linked to at least one nanoparticle; and (b) Adding an enhancement solution comprising silver ions and/or gold ions, and a reducing agent, whereby the silver ions and/or gold ions are reduced to metallic silver and/or gold which is deposited onto the surface of the at least one nanoparticle; (c) detecting the mass increase of the at least one nanoparticle using a SAW sensor.

Collodal gold (Au) and silver (Ag) particles and/or cluster complexes have been used in electron microscopy for labelling of target molecules. For example, cells that have such cluster complexes attached at a high density may appear dull red-purple in the microscope.

Immunogold silver staining (IGSS) was discovered in 1981 and has since then been used for signal amplification/enhancement to improve the sensitivity of immunodetection systems that function as an alternative to chemiluminescence and radionuclide labelling. IGSS make small sized and small amounts of a target molecule detectable for visual inspection by e.g. microscope (such as light, electron, scanning etc.) and colorimetric absorbance. The Immunogold silver staining technology is based on the presence of silver ions and a reducing agent such as e.g. hydroquinone, colloidal gold particles will act as catalysts to reduce silver ions to metallic silver. The silver is deposited onto the gold particles thereby enlarging the particles.

Some embodiments of the invention may use gold staining technology where the deposit covering a nanoparticle is acquired when gold ions in solution are catalytically deposited as metallic gold.

The increase in particle mass due to the deposits may in some embodiments of the invention be detected by a surface acoustic wave (SAW) device.

In some embodiments the invention relates to a method wherein the first molecular reaction component is attached to the surface of the SAW sensor. The surface of the SAW sensor should if not otherwise specified be understood as the entire surface or as a part of the surface. Part of the surface may be understood as the IDT and/or the reflector structures, and it may also be understood as the surface within the micro channels of the IDT and/or reflector structures, or on top of these structures, or both within the channels and on top of the IDT and/or reflector structures. The first molecular reaction component may be attached as a single component or it may form part of a network/grid located at the surface of the SAW sensor. For example the first molecular recognition component may be comprised in a hydrogel as disclosed in US application 20060024813.

In some embodiments the invention relates to a method wherein the second molecular reaction component linked to at least one nanoparticle, wherein the nanoparticle is a gold particle. The gold particle may itself function as a catalyst during the reduction of silver ions and/or gold ions to metallic silver and/or gold deposits. Each second molecular reaction component may dependent on the size and nature of both the component itself and the nanoparticle be linked to at least one nanoparticle. In some embodiments further molecular recognition components, optionally linked to at least one nanoparticle, may be included for the purpose of creating a network/grid structure and/or to amplify the detection signal of the method.

The nanoparticle according to the invention has a diameter of 0,05; 0,1; 0,2; 0,3; 0,4; 0,5; 0,6; 0,7; 0,8; 0,9; 1; 2; 3; 4; 5; 6; 7; 8; 9; 10; 11; 12; 13; 14; 15; 16; 17; 18; 19 or 20 nm.The diameter (size) and thus the mass of the nanoparticle may vary depending on the particular analysis set up and required sensitivity. There is a correlation between the nanoparticle diameter (size) and its mass (weight), for example the molecular weight of a gold particle with a diameter of 1 nm is 15.000 g/mol.

In some embodiments the invention relates to a method wherein the nanoparticle has a diameter from 0.05 nm to 20 nm.

For example a 10 nm size nanoparticle may increase in diameter to 300 nm within 5 minutes. Depending on the reaction time the diameter may increase to 500 nm or more.

The deposits made from suitable materials such as silver or gold provides a mass increase of the nanoparticle that is detectable by a surface acoustic wave (SAW) device. The development of metal deposits and thus the increase in particle diameter and mass may be stopped simply by removing the enhancement solution, for example by washing in pure water.

In one embodiment the invention furthermore relates to kit of parts comprising a microsensor for detecting an analyte in a sample, comprising: (a) a Surface Acoustic Wave sensor; comprising at least one first molecular recognition component immobilized to the surface of the SAW sensor; (b) at least one second molecular recognition component linked to at least one nanoparticle; (c) an enhancement solution comprising silver ions and/or gold ions; and (d) a reducing agent.

According to the method the silver ions and/or gold ions are reduced to metallic silver and/or gold which is deposited onto the surface of the at least one nanoparticle; wherein the mass increase of the at least one nanoparticle is detected by the SAW sensor.

### EXAMPLES

The following non-limiting examples serve to more fully describe the manner of using the above-described invention. It is understood that this example in no way serves to limit the scope of this invention, but rather are presented for illustrative purposes.

### EXAMPLE 1 - Assay of anti-mouse IgG/anti-HFABP mAb-ALP.

The procedure was performed according to the mode illustrated in Fig. 1.

A micro sensor consisting of two SAW resonator units (Fig. 1.-1, 2) was used. A SAW resonator unit three-dimensional surfaces (Fig. 1.-1) were coated with 20 nm of gold. An identical SAW resonator unit three-dimensional surfaces (Fig. 1.-2) were coated with SIO2. The gold surfaces of SAW resonator (Fig. 1.-1) were incubated with 100 ug/ml goat anti-mouse IgG, while the SIO2 surface of SAW resonator (Fig. 1.-2) were incubated with 100 ug/ml goat IgG.

The first step was adsorption of the anti-mouse antibody on the gold coated three-dimensional SAW surface. This was performed by equilibration of the gold/SAW resonator unit with 2 uL of 100 ug/mL antibody in PBS buffer solution for 2 hours in a humid environment. Both SAW resonator units were then washed three times with PBS buffer containing 0.05% tween. After this treatment the two SAW resonator units (Fig. 1-1, 2) was incubated with 1 % BSA in PBS for 1 hour at room temperature in a humid environment. Both SAW resonator units was then washed three times with TBS buffer/0.05% tween. The two SAW resonators units (Fig. 1-1, 2) were then exposed to varied concentrations of the mouse antibody (anti-HFABP mAb-ALP) label with an alkaline phosphatase enzyme (ALP) in PBS buffer for 15 minutes. After 3X wash with TBS/0.05% tween, BCIP/NBT (SIGMA) was added and the delta frequency between SAW resonator (Fig. 1-1) and SAW resonator unit (Fig. 2-2) was measured after 10 minutes (experiments I - VII).

### Control (experiment VIII) - Goat IgG was incubated on both SAW resonator units (Fig. 1-1,2)

The two SAW resonators units (Fig. 1-1, 2) were then exposed to 100 ng/ml of the mouse antibody (anti-HFABP mAb-ALP) label with an alkaline phosphatase enzyme (ALP) in PBS buffer for 15 minutes. After 3X wash with TBS/0.05% tween, BCIP/NBT (SIGMA) was added and the delta frequency between SAW resonator (Fig. 1-1) and SAW resonator unit (Fig. 2-2) was measured after 10 minutes (experiment VIII).

**TABLE I**

| **Experiment No.** | **anti-HFABP mAb-ALP (ng/ml)** | **SAW resonator unit (2)-(1) after 10 min. (kHz)** | |
|---|---|---|---|
| I | 100 | -2100 | |
| II | 100 | -2500 | |
| III | 100 | -2400 | |
| Mean | 100 | -2333 | CV=9% |
| IV | 33.3 | -1100 | |
| V | 10 | -350 | |
| VI | 10 | -325 | |
| VII | 10 | -175 | |
| Mean | 10 | -283 | C=33% |
| VIII (control) | control goat IgG | -1 | |

Using the data from table I a dose-response curve was generated and illustrated in Fig. 3. The R value = 1.00

### EXAMPLE 2 - Assay of anti-mouse IgG/anti-HFABP mAb-2ALP.

Same assay procedure as in Example I, however to further enhance the sensitivity an anti-mouse IgG/anti-HFABP mAb-2ALP was used as analyte (labelled with 2 ALP enzymes pr. Antibody).

**TABLE II**

| **Experiment No.** | **anti-HFABP mAb-ALP (ng/ml)** | **SAW resonator unit (2)-(1) after 10 min. (kHz)** | |
|---|---|---|---|
| I | 1 | -294 | |
| II | 1 | -310 | |
| III | 1 | -317 | |

### EXAMPLE 3 - Sensitivity test for G3 SAW resonator in combination with gold conjugated antibody.

Sensitivity testing protocol consist of three steps: A. Sensor cleaning procedure (Hellmanex-II treatment); B. Coding the SAW sensors with conjugates antibodies; and C. Measurement procedures.

### A. Hellmanex-II treatment Protocol

1. Prepare a 2 % Hellmanex-II solution:
2. Add 10 ml Hellmanex-II to 490 ml redistilled H₂O
3. SAW sensor surface tension is removed by using Hellmanex-II (2 %) for 30 min.
   at 37°C with gentle agitation 75 (rpm).
4. Add 200-250 ml 2 % Hellmanex solution into two big open glass containers.
5. Place 15-20 SAW sensors in each glass container.
6. Incubate at 30 minutes at 37°C with gentle agitation 75 (rpm).
7. Rinse with redistilled water 3x 100 ml,
8. Let sensors dry 15 minutes in an incubator at 37°C.
9. Store sensors until use in a petridish at RT (desiccated).

### B. Coding the SAW sensors with conjugates antibodies

1. Add 1 µl of a 400 ng/ml Anti-Mouse Gold conjugated antibody (Sigma G7652) into hole F2, leave F1 without any liquid.
2. Incubate for 2 hours in humid environment
3. Incubate sensors at 37°C for 15 minutes.
4. Store in dry environment in the refrigerator.

### C. Measurement procedures

1. Non-flow cartridge and G3 sensor is assemble and placed in the jig.
2. The measure program is started.
3. Run 5 minutes sensor stability test
4. Add 2 µl Silver enhancement solution (SPI 4180, LM silver enhancing kit) into both sensor holes (f1 and f2)
5. Reset the measuring program
6. Measure for 7 minutes.

### EXAMPLE 4 - Comparisons of sensitivity between Alkaline Phosphatase conjugated antibodies and gold conjugated antibodies.

Identical experiment was performed using Alkaline Phosphatase (ALP) conjugated antibodies and substrate solution: BCIP/NBT.

### A. Hellmanex-II treatment Protocol

Identical as described in example 3 above.

### B. Coding the SAW sensors with conjugates antibodies

Changes: Add 1 µl of a 400 ng/ml Mouse Alkaline phosphatase conjugated antibody into hole F2, leave F1 without any liquid.

### C. Measurement procedures

1. Non-flow cartridge and G3 sensor is assemble and placed in the jig.
2. The measure program is started.
3. Run 5 minutes sensor stability test
4. Add 2 µl BCIP/NBT solution (Sigma B3679) into both sensor holes (f1 and f2)
5. Reset the measuring program
6. Measure for 15 minutes.

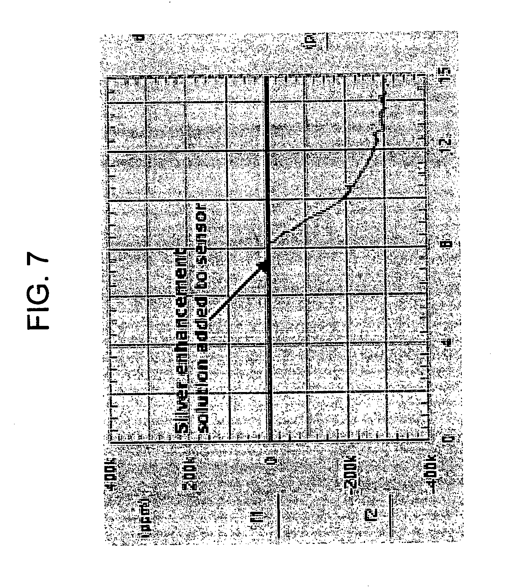

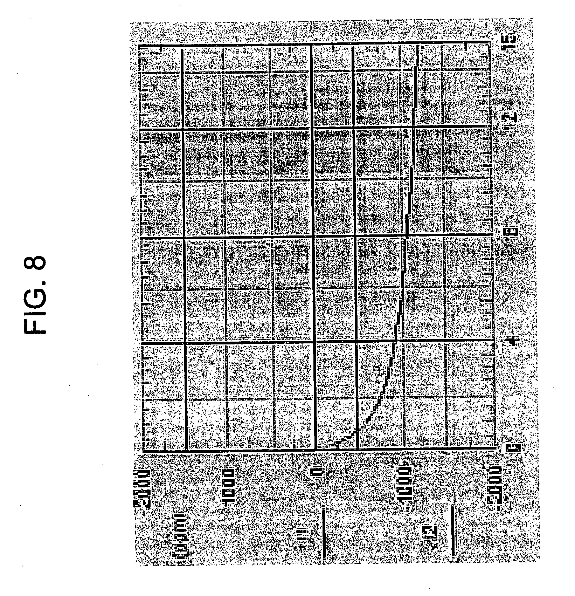

From sensitivity point of view it seems likely that the gold method gives a 300x increased signal compared to our ALP method judged by Fig. 7 (gold method) and Fig. 8 (Alkaline phosphatase method).

### Visual comparisons between the two methods (gold conjugated and alkaline phosphatase conjugated antibody method) - Conclusion.

An improvement of the signal amplitude has recently been invented by Atonomics. Until now Alkaline Phosphatase (ALP) conjugated antibodies have been used for signal generation on the oscillating SAW sensors. Several disadvantages have been identified using this ALP method such as:

ALP conjugated antibody binds unspecifically to the gold and resin wall inside the sensor cavity and on the surface of the substrate. Such binding creates non specific BCIP/NBT precipitate and eventually give rice to an increase of the CV value.

The BCIP/NBT precipitate in the SAW micro channels and on top of the SAW structures are not distributed as a plane layer all over the sensor structure (due to oscillating nature of the SAW sensor), but in a more random way on the sensor surface as illustrated at Fig. 4B - this also could give rice to increase of the CV value.

Since the Alkaline phosphatase method is an enzymatic process it can loose its activity over time due to temperature and handling issues. Also this could give rice to increase of the CV value.

To overcome such disadvantages Atonomics has initiated the test of nano-gold conjugated antibodies instead of Alkaline phosphatase conjugated antibodies.

From sensitivity point of view it seems likely that the gold method gives a 300X increased signal compared to our ALP method judged by Fig. 2 and Fig. 3.

**TABLE III: A comparison between the ALP method and the gold conjugated method**

| | **ALP conjugated antibody/BCIP/NBT method** | **New nano-gold conjugated antibody/silver enhancement method** |
|---|---|---|
| **SAW signal (sensitivity)** | Present standard | 100-1000X more SAW signal |
| **Generate signal in form of:** | Generate BCIP/NBT precipitate in a more random fashion on the SAW sensor surface | Generate a uniform layer of metal Ag on the SAW sensor surface |
| **Non specific binding (reproducibility)** | The BCIP/NBT precipitate from the wall of the cavity can create non specific signal | A silver layer is also generated on the gold wall in the cavity, however it does not interfere with the assay |
| **Stability issue (reproducibility and sensitivity)** | Enzymatic reaction | Non enzymatic reaction and thereby more stable. |
| **IP issue** | Patent application pending | Patent application pending |

## Claims

1. Method for detecting a target analyte in a sample, said method comprising the steps:
(a) reacting the analyte in a sample with a first molecular recognition component and a second molecular recognition component linked to at least one nanoparticle; and
(b) adding an enhancement solution comprising silver ions and/or gold ions, and a reducing agent, whereby the silver ions and/or gold ions are reduced to metallic silver and/or gold which is deposited onto the surface of the at least one nanoparticle; and
(c) detecting the at least one nanoparticle comprising deposited silver ions and/or gold ions by a SAW sensor.

2. Method according to claim 1, wherein the nanoparticle is a gold particle.

3. Method according to any of the claims 1 or 2, wherein the nanoparticle has a diameter from 0.05 nm to 20 nm.

4. Method according to claim 3, wherein the nanoparticle has a diameter of 0,05; 0,1; 0,2; 0,3; 0,4; 0,5; 0,6; 0,7; 0,8; 0,9; 1; 2; 3; 4; 5; 6; 7; 8; 9; 10; 11; 12; 13; 14; 15; 16; 17; 18; 19 or 20 nm

5. Method according to any of the claims 1-4, wherein an about 10 nm size nanoparticle increases in diameter to about 300 nm within 5 minutes.

6. Kit of parts comprising a microsensor for detecting an analyte in a sample, comprising: (a) a Surface Acoustic Wave sensor; said Surface Acoustic Wave sensor comprising at least one first molecular recognition component immobilized to the surface of the SAW sensor;
(b) at least one second molecular recognition component linked to at least one nanoparticle;
(c) an enhancement solution comprising silver ions and/or gold ions; and
(d) a reducing agent.

## Patentansprüche

1. Verfahren zur Detektion eines Zielanalyten in einer Probe, wobei das Verfahren die Schritte umfasst:
(a) Reaktion des Analyten in einer Probe mit einer ersten Komponente zur molekularen Erkennung und einer zweiten an mindestens ein Nanopartikel gebundenen Komponente zur molekularen Erkennung; und
(b) Zugabe einer Verstärkungslösung enthaltend Silberionen und/oder Goldionen und ein Reduktionsmittel, wobei die Silberionen und/oder Goldionen zu metallischem Silber und/oder Gold reduziert werden, das auf der Oberfläche des mindestens einen Nanopartikels abgeschieden wird; und
(c) Detektion des mindestens einen Nanopartikels enthaltend abgeschiedene Silberionen und/oder Goldionen mit einem SAW Sensor.

2. Verfahren nach Anspruch 1, worin das Nanopartikel ein Goldpartikel ist.

3. Verfahren nach einem der Ansprüche 1 oder 2, worin das Nanopartikel einen Durchmesser von 0, 05 nm bis 20 nm hat.

4. Verfahren nach Anspruch 3, worin das Nanopartikel einen Durchmesser von 0,05; 0,1; 0,2; 0,3; 0,4; 0,5; 0,6; 0,7; 0,8; 0,9; 1; 2; 3; 4; 5; 6; 7; 8; 9; 10; 11; 12; 13; 14; 15; 16; 17; 18; 19 oder 20 nm hat.

5. Verfahren nach einem der Ansprüche 1 - 4, worin ein Nanopartikel von etwa 10 nm Größe im Durchmesser auf 300 nm innerhalb von 5 Minuten wächst.

6. Kit-of-Parts enthaltend einen Mikrosensor zur Detektion eines Analyten in einer Probe, enthaltend: (a) einen Oberflächenwellensensor; wobei der Oberflächenwellensensor mindestens eine erste Verbindung zur molekularen Erkennung enthält, aufgebracht auf die Oberfläche des SAW Sensors;
(b) mindestens eine zweite Verbindung zur molekularen Erkennung gebunden an mindestens ein Nanopartikel;
(c) eine Verstärkungslösung enthaltend Silberionen und/oder Goldionen; und
(d) ein Reduktionsmittel.

## Revendications

1. Procédé pour détecter un analyte cible dans un échantillon, ledit procédé comprenant les étapes :
(a) réaction de l'analyte dans un échantillon avec un premier composant de reconnaissance moléculaire et un second composant de reconnaissance moléculaire lié à au moins une nanoparticule ; et
(b) addition d'une solution d'amplification comprenant des ions argent et/ou des ions or, et un agent réducteur, où les ions argent et/ou les ions or sont réduits en argent métallique et/ou or métallique qui est déposé sur la surface de la au moins une nanoparticule ; et
(c) détection de la au moins une nanoparticule comprenant des ions argent et/ou des ions or déposés par un capteur OAS.

2. Procédé selon la revendication 1, où la nanoparticule est une particule d'or.

3. Procédé selon l'une quelconque des revendications 1 ou 2, où la nanoparticule a un diamètre de 0,05 nm à 20 nm.

4. Procédé selon la revendication 3, où la nanoparticule a un diamètre de 0,05 ; 0,1 ; 0,2 ; 0,3 ; 0,4 ; 0,5 ; 0,6 ; 0,7 ; 0,8 ; 0,9 ; 1 ; 2 ; 3 ; 4 ; 5 ; 6 ; 7 ; 8 ; 9 ; 10 ; 11 ; 12 ; 13 ; 14 ; 15 ; 16 ; 17 ; 18 ; 19 ou 20 nm.

5. Procédé selon l'une quelconque des revendications 1-4, où une nanoparticule d'une taille d'environ 10 nm augmente en diamètre à environ 300 nm en 5 minutes.

6. Kit de parties comprenant un microcapteur pour détecter un analyte dans un échantillon, comprenant : (a) un capteur d'ondes acoustiques de surface ; ledit capteur d'ondes acoustiques de surface comprenant au moins un premier composant de reconnaissance moléculaire immobilisé à la surface du capteur OAS ;
(b) au moins un second composant de reconnaissance moléculaire lié à au moins une nanoparticule ;
(c) une solution d'amplification comprenant des ions argent et/ou des ions or ; et
(d) un agent réducteur.
